# EUROPEAN PATENT APPLICATION

(11) **EP 4 667 570 A1**
(43) Date of publication of application: **24.12.2025**
(21) Application number: 24183463.9
(22) Date of filing: 20.06.2024
(51) Int. Cl.: C12N 15/113

(54) **NUCLEIC ACID MOLECULE COMPLEX FOR TARGET PSEUDOURIDYLATION IN MAMMALIAN CELLS**

(71) Applicant: Johannes Gutenberg-Universität Mainz, 55122 Mainz (DE)
(72) Inventor: SCHARTEL, Lukas, 55116 Mainz (DE); LEMKE, Edward, 55122 Mainz (DE)
(74) Representative: Patentanwälte Dr. Keller, Schwertfeger Partnerschaft mbB

(57) **Abstract**

The invention relates to a nucleic acid molecule complex for pseudouridylation of a target RNA in a mammalian cell comprising a guide RNA (gRNA) that at one end is flanked by a first twister ribozyme and at its other end by a second twister ribozyme to generate a circular gRNA, wherein the nucleic acid molecule complex has the capability to generate circular gRNA which is characterized by a secondary structure comprising two pseudouridylation pockets that harbor antisense guide sequences. More specifically, the first twister ribozyme is a P3 twister ribozyme and the second twister ribozyme is a P1 twister ribozyme, wherein each twister ribozyme is separated from the gRNA by a linker. In a further aspect, the invention relates to a method for generating circular gRNA. The invention furthermore relates to a nucleic acid molecule complex as defined herein for use in the treatment of a genetic disease caused by a premature stop codon. It furthermore relates to a pharmaceutical composition, and a delivery vector to express the construct in mammalian cells to generate circular gRNA.

## Description

### Field of the Invention

The present invention relates to the field of targeted pseudouridylation, specifically guide RNA that selectively modifies RNA for the treatment of a genetic disease caused by premature stop codons.

### Background of the Invention

Pseudouridylation is the process of converting uridine (U) to pseudouridine (Ψ) in RNA molecules. This is the most abundant RNA modification and involves the isomerization of uridine, where the uracil base is attached to the ribose via a carbon-carbon (C-C) bond instead of the usual nitrogen-carbon (N-C) bond. This structural change significantly enhances the stability and function of RNA molecules. The presence of Ψ strengthens hydrogen bonding and stacking interactions, which stabilizes the RNA's three-dimensional structure. In ribosomal RNA (rRNA), pseudouridylation is crucial for maintaining the proper structure and function of the ribosome. It enhances the accuracy of protein synthesis by promoting correct codon-anticodon pairing during translation. In transfer RNA (tRNA), Ψ contributes to the correct folding and stability of the tRNA molecule, ensuring efficient translation. Pseudouridylation in small nuclear RNAs (snRNAs) is essential for the proper function of the spliceosome, the molecular machinery responsible for splicing pre-mRNA. This modification ensures the accurate removal of introns and the joining of exons, which is critical for mRNA maturation. Pseudouridine modifications can influence the processing and intracellular localization of RNA molecules, affecting their stability and function within different cellular compartments

Pseudouridylation is catalyzed by pseudouridine synthases, a family of enzymes that recognize specific uridine residues in RNA and convert them to pseudouridine. There are different pseudouridine synthases for different types of RNA, and their activity is often guided by small RNA molecules, such as small nucleolar RNAs (snoRNAs), which direct the enzymes to the correct modification sites and is therefore called guide RNA (gRNA). Genetic diseases that arise from premature stop codons are caused by mutations within the DNA sequence, which introduce an erroneous stop signal within the coding region of an mRNA. This early stop signal results in the production of a truncated and usually non-functional protein, leading to various disorders depending on the protein affected. Conditions such as cystic fibrosis and Duchenne muscular dystrophy are examples where premature stop codons play a central role in disease pathology.

Pseudouridylation offers a promising approach to address these genetic disorders. This process involves the enzymatic modification of specific uridine residues in RNA, converting them into pseudouridine. For treating genetic diseases, pseudouridylation can be used to modify mRNAthat contains premature stop codons. The introduction of pseudouridine at specific sites near or at the stop codon can affect the mRNA's interaction with the ribosomal machinery during translation. Remarkably, pseudouridine can induce the ribosome to read through a premature stop codon, continuing the translation process instead of halting prematurely. This read-through allows to produce a full-length, potentially functional protein, thus alleviating the deficiency caused by the truncated protein.

The application of pseudouridylation in this manner harnesses the cell's natural translation machinery to override faulty stop signals, offering a therapeutic strategy that could restore normal protein function in patients with genetic diseases caused by premature stop codons. This innovative approach not only highlights the potential of RNA modifications in treating genetic disorders but also opens new avenues for research and development in genetic therapy.

EP 377 521 0 B1 describes a nucleic acid molecule for pseudouridylation of a target uridine in a target RNA in a mammalian cell. The nucleic acid molecule consisting of a single guide region corresponding to one or two hairpin structures of wild-type H/ACA snoRNA. The nucleic acid molecule can form a partially double-stranded nucleic acid complex with a target RNA comprising the target uridine, wherein the partially double-stranded nucleic acid complex can contact a mammalian pseudouridylation enzyme, wherein the guide region assists in positioning the target uridine in the partially double-stranded nucleic acid complex to be converted to a pseudouridine by the mammalian pseudouridylation enzyme.

Adachi et al. describe targeted PTC pseudouridylation to nonsense suppression. Targeted PTC pseudouridylation is achieved by introducing into cells a designer box H/ACA RNA targeting the mRNA PTC. Conversion of the uridine to pseudouridine at the PTC suppresses NMD and promotes PTC readthrough, restoring full-length protein (Adachi H, Pan Y, He X, Chen JL, Klein B, Platenburg G, Morais P, Boutz P, Yu YT. Targeted pseudouridylation: An approach for suppressing nonsense mutations in disease genes. Mol Cell. 2023 Feb 16;83(4):637-651.e9. doi: 10.1016/j.molcel.2023.01.009. Epub 2023 Feb 9. PMID: 36764303; PMCID: PMC9975048).

Although nucleic acid molecules for pseudouridylation of a target uridine in a target RNA are known, there is still an unmet medical need for a nucleic acid molecule complex for pseudouridylation of a target RNA in a mammalian cell with improved selectivity and efficacy.

### Description of the Invention

Against this background, it is the object of the present invention to provide an improved nucleic acid molecule complex for pseudouridylation to increase efficacy and selectivity in targeted pseudouridylation in mammalian cells.

The solution of this problem is provided by nucleic acid molecule complexes for pseudouridylation as defined in the following claims. Advantageous embodiments of the invention are formulated in the dependent claims.

A "nucleic acid molecule complex" as used herein refers to a genetic construct that is composed of guide RNA (gRNA) and two flanking twister ribozymes, preferably selected from the group consisting of P1/P3 twister ribozymes.

This invention is based on the surprising finding that a combination of twister ribozymes and gRNA leads to a circularization of the construct forming a circular gRNA structure, which makes the targeted pseudouridylation, i.e. the modification of target RNA with pseudouridine, in mammalian cells more efficient. In order to produce circular gRNA, it is required that the gRNAs possess a common secondary structure with characteristic pseudouridylation pockets that harbor antisense guide sequences.

More specifically, the nucleic acid molecule complex of the present invention leads to a circularized gRNA that is characterized by two loops, each loop bearing a pseudouridylation pocket followed by a linker and a ligation stem. The ligation stem is a remnant of the ribozymes after cleavage. In three dimensions, the gRNA, the linker and the ligation stem form a Y-like shape. The secondary structure of the inventive circular gRNAs is characterized in that there are two bubbles or pockets formed in each of the two RNA branches that are formed upwards in the Y-shaped molecule. These pockets bear the anti-sense nucleic acid sequences required to target the mRNAs for pseudouridylation. Most importantly, the nucleic acid sequences that are part of the pseudouridylation pockets are not complementary to each other so that the two bubbles can be formed. Due to the secondary structure, the two pockets are sterically angled away from each other.

As shown herein, circular gRNA significantly enhances the effectiveness of targeted pseudouridylation as compared to linear gRNA commonly used in the field. The invention provides an efficient way to generate highly selective circular gRNA molecules by combining gRNA bearing the target mRNA sequence and at least two twister ribozymes as well as non-complementary RNA sequences that are non-complementary to each other such that two pseudouridylation pockets can be formed in the generated circular gRNA molecule. Because pseudouridylation is more effective, the nucleic acid molecule complex of the invention is suitable for use in the treatment of a genetic disease caused by premature stop codons.

One further advantage of the gRNA construct according to the present invention is that it is more stable, because degradation by exonucleases is less due to the lack of 5' - and 3' - ends. It can therefore be assumed that the circular gRNA accumulates to a greater extent in the cell leading to a higher expression rate.

In a first aspect, the invention therefore relates to a nucleic acid molecule complex for pseudouridylation of a target RNA in a mammalian cell comprising a guide RNA (gRNA) that at one end is flanked by a first twister ribozyme and at its other end by a second twister ribozyme to generate circular gRNA, wherein the nucleic acid molecule complex has the capability to generate circular gRNA, wherein the circular gRNA is characterized by a secondary structure comprising two pseudouridylation pockets that harbor antisense guide sequences.

Usually, circular gRNA is generated within a controlled cellular environment. For example, circular gRNA is generated when the nucleic acid molecule complex is expressed in a mammalian cell. Alternatively, a cell-free system can be used that resembles a living cell in an in vitro environment.

The gRNA comprises a nucleic acid sequence that is complementary to a target mRNA sequence for pseudouridylation. The sequence includes a uridine residue designated for modification. The complementary sequence is critical to ensure that the nucleic acid molecule complex specifically binds to the target mRNA. In order to obtain circular gRNA, the inventive gRNAs are characterized by a common secondary structure forming pseudouridylation pockets that harbor the antisense guide sequences.

In a preferred embodiment, the first twister ribozyme is a P3 twister ribozyme and the second twister ribozyme is a P1 twister ribozyme, which are able to form the pseudouridylation pockets in the generated circular gRNAs.

The P1 and P3 twister ribozyme variants belong to a class of naturally occurring small self-cleaving RNA enzymes (ribozymes). These ribozymes perform a specific biological function by catalyzing the cleavage of a phosphodiester bond within their own RNA sequence. The ability to self-cleave allows the ribozyme to regulate gene expression by controlling the stability and availability of RNA molecules in which they are embedded. In the construct of the present invention, the P1/P3 twister ribozymes are located upstream and downstream of the gRNA.

To prevent steric hindrance and to increase flexibility, the two twister ribozymes are preferably separated from the gRNA by a linker nucleic acid sequence (in short "linker"). One type therefore relates to a construct that has the 5' to 3' structure of P3 twister ribozyme - gRNA - P1 twister ribozyme. Another variant relates to a construct consisting of the 5' to 3' structure of P1 twister ribozyme - gRNA - P3 twister ribozyme. Both structures result in highly-selective and efficient circular gRNA when expressed in living mammalian cells.

Preferably, the linker is a polyadenine linker (poly(A) linker). Preferably, the poly(A) linker sequence comprises 2 to 90 nucleotides, preferably 5 to 20 nucleotides, more preferably 7 to 12 nucleotides. However, it is apparent that the invention covers any length and types of linkers, and also covers intermediate ranges of linker lengths that are not expressively listed here.

In one specific embodiment, the linker is a poly(A) linker flanking the gRNA sequence at its 5' end and its 3' end. In this embodiment, one linker is positioned after the nucleic acid sequence of the P3 twister ribozyme and the other linker is positioned before the nucleic acid sequence of the P1 twister ribozyme. The linker sequences that are linked to the gRNA sequence can have different lengths or be identical in length, and can comprise the same or different nucleic acid sequence. In a preferred embodiment, the linkers each comprise a poly(A) sequence with a length of about 10 nucleotides.

In alternative embodiments, the linker arranged before and after the gRNA sequence is selected from the group consisting of poly(U) linker, poly(T) linker, poly(G) or poly(C) linker, random sequence linker, spacer elements, flexible nucleotide linker, peptide nucleic acids (PNAs) or bridging molecules.

In a preferred embodiment, the nucleic acid molecule complex further comprises a promoter, preferably a U6 promoter, to express a 5' - P3 twister - gRNA - P1 twister - 3' molecule.

It is apparent that any suitable promoter will work in the context of the present invention. Preferably, the promoter is an RNA polymerase III promoter that drives expression of the downstream twister ribozyme and gRNA sequences. In alternative embodiments the promoter can be a H1 promoter, CMV promoter (Cytomegalovirus Promoter), EF1α promoter (Elongation Factor 1-alpha), T7, T3, SP6 promoter, synthetic or artificially optimized promoters.

In a preferred embodiment, the gRNA flanked by the twister ribozymes is H/ACA box small nucleolar RNA (H/ACA snoRNA). Small nucleolar RNAs (snoRNAs) are a class of small RNA molecules that primarily function in the chemical modification of other RNAs, particularly ribosomal RNA (rRNA) within the nucleolus of eukaryotic cells. These H/ACA box RNAs possess a common secondary structure with characteristic pseudouridylation pockets that harbor antisense guide sequences. As shown herein, these pockets can be further engineered to complement any target RNA, such that it can be used in a programmable pseudouridine-dependent RNA-modifying organelle.

Circularization of the gRNA can be carried out in vitro by any suitable method known in the art. Alternatively, in vivo expression of the construct in infected or transformed cells is preferred to generate circularized gRNA. After expression of the gRNA/ribozyme complex in living cells, the twister ribozymes cleave themselves, leaving an RNA stem that is ligated by the endogenous RNA ligase RtcB. Circularization of the resulting gRNAs in cells can be confirmed, for instance, by using RT-PCR with divergent primer pairs where only a circular gRNA results in a PCR product.

In some embodiments, the gRNA used in the context of the present invention comprises a complementary sequence to a target mRNA sequence for pseudouridylation, structural/docking sites, which may bind to proteins or other molecules that are part of the editing complex and additional functional sequences that promote their stability, localization, or processing within the cell.

In a preferred embodiment, the nucleic acid molecule complex further comprises one or more short aptamer sequences of RNA loops that are arranged after or before the two twister ribozymes. Aptamers in context of the present invention relate to single-stranded RNA molecules that can bind to specific ligands, including proteins, small molecules, and other nucleic acids, with high affinity and specificity. RNA loops or aptamers are designed to recognize and bind to specific molecular targets. This binding can enhance the specificity of the nucleic acid molecule complex, directing it to a specific location within the cell or bringing it into proximity with a particular biomolecule necessary for its function. For example, the aptamers can be used to recruit and stabilize pseudouridine synthase enzymes at the desired modification site on the mRNA. This enhances the efficiency of the modification process and ensures that it occurs precisely where needed.

In preferred embodiments, the short aptamer sequences are selected from the group consisting of a ms2 loop, boxb, pp7 or Qbeta loop. A preferred short aptamer sequence used in the construct of the invention forms a ms2 loop, which is able to bind to MCP protein such that the nucleic acid molecule complex can be recruited to a specific place within the cell. In a preferred embodiment, the RNA loop is located at the beginning of or is part of the linker sequence.

As shown herein, coupling the circular gRNA complex to a gRNA optimized RNA editing organelles (OREO) is in particular advantageous in order to increase efficiency and selectivity of the inventive construct.

In a further aspect of the present invention, the nucleic acid molecule complex interacts via the short aptamer sequences of RNA loops with an optimized RNA editing organelle (OREO), comprising
a. a plasma membrane anchor,
b. an intrinsically disordered region (IDR), and
c. a major capsid protein (MCP) serving as a recruitment domain for the nucleic acid molecule complex.

In a preferred embodiment, the nucleic acid molecule complex of the invention comprises an OREO or parts thereof as described herein.

OREOs are synthetic organelles (often also called "designer organelles") that are fundamentally constructed to optimize specific biochemical pathways that are otherwise inefficient or non-existent in natural biological systems. By engineering these specialized compartments, one can target and modify biological molecules with unprecedented precision, reducing off-target effects and enhancing cellular function. An OREO according to the present invention is specifically engineered to carry out targeted RNA pseudouridylation within a controlled cellular environment.

The design of these organelles involves assembling various molecular components that function synergistically. The plasma membrane anchor facilitates the localization of the organelle within specific cellular compartments, ensuring that the biochemical processes are carried out in the desired cellular locale. Additionally, the IDR contributes to the structural framework of the organelle. This region allows for the necessary flexibility and dynamic interaction with other cellular components, essential for the organelle's functionality.

In a preferred embodiment, the plasma membrane anchor of the OREO consists of a N-terminal domain of LCK tyrosine kinase and the IDR is derived from the protein FUS.

The MCP's role as recruitment domain in the OREO is crucial as it enables the selective recruitment of specific RNA molecules or proteins, enhancing the organelle's specificity and efficiency in processing its target molecules. These components are often fused with functional proteins like dyskerin, a pseudouridine synthase, under the control of a CMV promoter. The use of dyskerin is particularly remarkable as it mediates the pseudouridylation process, converting specific uridine residues in the target RNA into pseudouridine. This modification can significantly alter the RNA's stability and function, which is vital in genetic regulation and expression.

Preferably, the MCP is genetically engineered to include a fusion tag that binds specifically to ms2 loops. Ms2 loops can be used to recruit modifying enzymes such as pseudouridine synthases to specific RNA targets.

In some embodiments, the MCP is N-terminally fused to pseudouridine synthase dyskerin, preferably to a truncated version of dyskerin (22-424). The truncated version lacks the NLS/NoLS signals at the N or C terminus and further includes a nuclear export signal at the N terminus. In an alternative embodiment, the dyskerin is isoform 3. Dyskerin is a component of the H/ACA box snoRNA complex that mediates pseudouridylation of ribosomal RNA. In alternative embodiments, synthetic or engineered pseudouridine synthases can be used.

OREOs according to the present invention are constructed based on the principles of phase separation, a crucial technique in creating artificial, membrane-less organelles within mammalian cells. This approach allows for the spatial and temporal control of biochemical reactions, a fundamental aspect when dealing with intricate cellular mechanisms.

In a further aspect, the invention also refers to a method, preferably an in vitro method, for converting a uridine in a target RNA molecule into a pseudouridine, comprising the steps of:
- providing a target RNA molecule containing at least one uridine residue,
- providing a pseudouridine synthase enzyme,
- synthesizing a circular gRNA that includes sequences complementary to regions flanking the uridine residue in the target RNA,
- incubating the target RNA with the circular gRNA and the pseudouridine synthase under conditions suitable for binding and modification,
- and detecting the conversion of uridine to pseudouridine using mass spectrometry or nucleoside analysis,
wherein the circular gRNA used is generated by using a nucleic acid molecule complex as described herein.

In a further aspect, the invention relates to a method for generating circular gRNA, comprising the steps:
- providing a nucleic acid molecule complex as defined herein,
- transferring the nucleic acid molecule complex into a mammalian cell,
- expressing the nucleic acid molecule complex in said mammalian cell to produce circular gRNA.

The so produced circular gRNA is characterized by a secondary structure comprising two pseudouridylation pockets that harbor antisense guide sequences.

Preferably, human cells are transfected with a nucleic acid molecule complex consisting of U6 promoter and gRNA flanked by the twister ribozymes. After expression, the twister ribozymes cleave themselves resulting in an RNA stem that contains the desired circular gRNA.

In preferred embodiments, transferring the nucleic acid molecule complex into a mammalian cell is carried out by any suitable method described herein preferably by transformation, transfection, or transduction. The use of a delivery vector as defined herein is preferred.

The nucleic acid molecule complex designed for pseudouridylation of a target RNA in mammalian cells can be effectively used to treat genetic diseases caused by premature stop codons. This complex specifically modifies certain uridine residues in mRNA to pseudouridine, which can potentially suppress premature stop codons during the translation process. This modification allows the ribosome to read through the premature stop codon, leading to the production of a full-length, functional protein. By restoring the correct synthesis of proteins that are otherwise truncated due to genetic mutations, this approach can mitigate the effects of the disease, offering a novel therapeutic strategy for conditions such as cystic fibrosis, Duchenne muscular dystrophy, and other similar genetic disorders.

In a further aspect, the invention therefore relates to the nucleic acid molecule complex for pseudouridylation of a target RNA in a mammalian cell is for use in the treatment of a genetic disease caused by premature stop codons. Stop codon suppression by pseudouridine offers great potential in the treating these diseases or conditions. The enhancing efficiency of circularized gRNA (in particular circularized H/ACA box-derived snoRNAs) holds the potential for targeting the aforementioned diseases in the future.

The genetic diseases caused by premature stop codons is selected from the group consisting of cystic fibrosis, Duchenne muscular dystrophy (DMD), Beta-thalassemia, Hurler syndrome, Usher syndrome, Ataxia-Telangiectasia (AT), Retinitis pigmentosa.

The treatment of genetic diseases caused by premature stop codons is achieved by promoting pseudouridylation at specific stop codon sites in the target mRNA, thereby effectively extending translation and preventing mRNA degradation.

In a further aspect, the present invention relates to a pharmaceutical composition comprising a nucleic acid molecule complex as defined herein, and one or more of a pharmaceutically acceptable carrier, stabilizer or solvent.

In preferred embodiments, the pharmaceutical composition is formulated for administration via injection, infusion, or direct organ delivery, and intended for the treatment of genetic disorders involving mRNA misprocessing or premature stop codons.

A preferred pharmaceutical composition of the present invention is formulated in a way that fits to its intended route of administration. A pharmaceutical composition can include a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose.

In some embodiments, the pharmaceutical composition is adjusted for injecting directly into the patient and includes at least one viscosity enhancing agent and/or at least one preservative. Pharmaceutical compositions suitable for injectable use include sterile aqueous solutions (where water soluble) or dispersions and sterile powders for the extemporaneous preparation of sterile injectable solutions or dispersion. In some embodiments, the carrier is a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In some embodiments, it will be preferable to include isotonic agents, for example, sugars, poly alcohols such as mannitol, sorbitol, sodium chloride in the composition. Prolonged absorption of the injectable compositions can be brought about by including in the composition an agent, which delays absorption, for example, aluminum monostearate and gelatin.

Sterile injectable solutions can be prepared by incorporating the active compound in the required amount in an appropriate solvent with one or a combination of ingredients enumerated above, as required, followed by filter sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

In a further aspect, the present invention relates to a delivery vector for pseudouridylation of a target RNA in a mammalian cell, comprising a nucleic acid molecule complex as described herein, in particular a gRNA/ribozyme complex that is converted in circular gRNA when expressed in mammalian cells, in particular human cells.

The nucleic acid molecule complex can be applied for pseudouridylation in a cell by any suitable method. In some embodiments, the construct can be delivered to the cell (either in vitro, ex vivo or in vivo) is by using a delivery vehicle such as a viral vector. One preferred viral vector is based on adeno-associated virus (AAV) or helper-dependent adenovirus (HdAd). Another preferred viral vector is for instance a retroviral vector such as a lentivirus vector and the like. In alternative embodiments, plasmids or artificial chromosomes usable for targeted homologous recombination and integration in the human genome of cells may be suitably applied for delivery of a nucleic acid molecule complex as defined herein.

In alternative embodiments, introduction of the nucleic acid molecule complex into the host cell can occur e.g. by transformation, transfection, or transduction.

To achieve sufficient intracellular concentrations of antisense molecules, vector constructs in which the nucleic acid molecule complex is placed under the control of a strong polymerase II or polymerase III promoter are preferred. In a preferred embodiment, the promoter for expressing the nucleic acid molecule complex is a U6 promoter.

One preferred type of delivery vector is a plasmid. Another type of delivery vector is a viral vector, wherein additional nucleic acid segments can be ligated into the viral genome. The invention covers all suitable types of vectors. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, expression vectors are capable of directing the expression of genes to which they are operatively linked.

In preferred embodiments, solutions for injection of the nucleic acid molecule complex or delivery vector, include, but are not limited to comprise a sterile diluent such as water for injection, saline solution, fixed oils, glycerine, polyethylene glycols, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The pharmaceutical preparation is enclosed in vials, ampoules, disposable syringes or multiple dose vials made of glass or plastic.

In a preferred embodiment, the nucleic acid molecule complex or delivery vector is provided in water containing sucrose, hydrochloric acid, sodium hydroxide ethanol, sodium chloride, I-histidine, tris, magnesium chloride, polysorbate 80, and EDTA. Preferably, the medium is A195, which is a Tris-buffered liquid adenovirus formulation comprising 10 mM Tris, 10 mM histidine, 75 mM NaCl, 5 % sucrose (w/v), 1 mM MgCl2, 0.02% (w/v), PS-80, 0.1 mM EDTA, 0.5% ethanol (v/v), pH 7.4.

In a further aspect, the present invention relates to a mammalian cell expressing a nucleic acid molecule complex as defined herein, or a delivery vector as described herein. Preferably, the mammalian cell is a human cell.

In some embodiments, the present invention relates to a mammalian cell, comprising:
- a stably integrated synthetic organelle designed for the spatially confined pseudouridylation of mRNA,
- the organelle comprising a scaffold of intrinsically disordered proteins and membrane anchoring domains,
- and a set of engineered nucleic acids encoding gRNAs and pseudouridine synthase, specifically targeting and modifying uridine residues within mRNA transcripts associated with disease phenotypes, thereby enhancing the cell's capability to produce functional proteins from otherwise defective mRNAs,
wherein the gRNAs are circular gRNAs that are generated by a nucleic acid molecule complex comprising a guide RNA (gRNA) that at one end is flanked by a P3 twister ribozyme and at its other end by a P1 twister ribozyme, wherein the gRNA is optionally separated from the twister ribozymes by linker sequences.

In conclusion, the development of a nucleic acid molecule complex for pseudouridylation of a target RNA in a mammalian cell offers a promising therapeutic strategy for the treatment of genetic diseases caused by premature stop codons, with higher efficacy and specificity.

It will be apparent for a person skilled in the art that the invention can be further improved or further characterized by the features described herein or advantageous embodiments, each of which is advantageous in itself and can be combined with one another as desired.

### Short Description of the Figures

Figure 1 shows that circular gRNA of the invention allow enhanced pseudouridine-mediated stop-codon suppression.
Figure 2 shows that circular gRNA of the invention increase designer organelle-mediated pseudouridylation.
Figure 3 shows that circular gRNA of the invention tagged with an ms2 loop enhance selectivity.

### Examples

The present disclosure is further illustrated by the following non-limiting examples, the teachings of which can be generalized and combined with the foregoing disclosure.

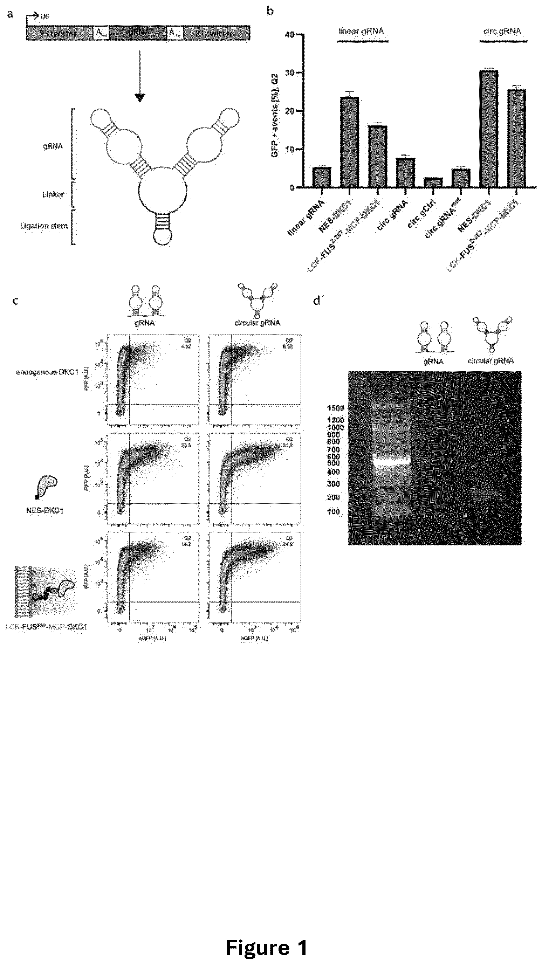

**Figure 1** illustrates that circular gRNAs allow enhanced pseudouridine-mediated stop-codon suppression. Figure 1a shows a cartoon depicting the generation of circular gRNAs from a U6 promoter using the Tornado system. A 10-nt polyadenine linker was incorporated 5' and 3' of the gRNA to maintain flexibility. Figure 1b shows the circular gRNAs outperform linear gRNAs in HEK293T cells co-transfected with the efficiency reporter and DKC1 constructs. The SEMs of three independent measurements are shown, in which 5 × 104 live cells were measured using flow cytometry. Figure 1c illustrates flow cytometry scatter plots shown for key samples in b. d, Circularization of gRNAs was confirmed by RT-PCR, expected product size is 199bp, purified PCR samples were run on an 1.8% agarose gel (left line shows a DNA ladder given in base pair units).

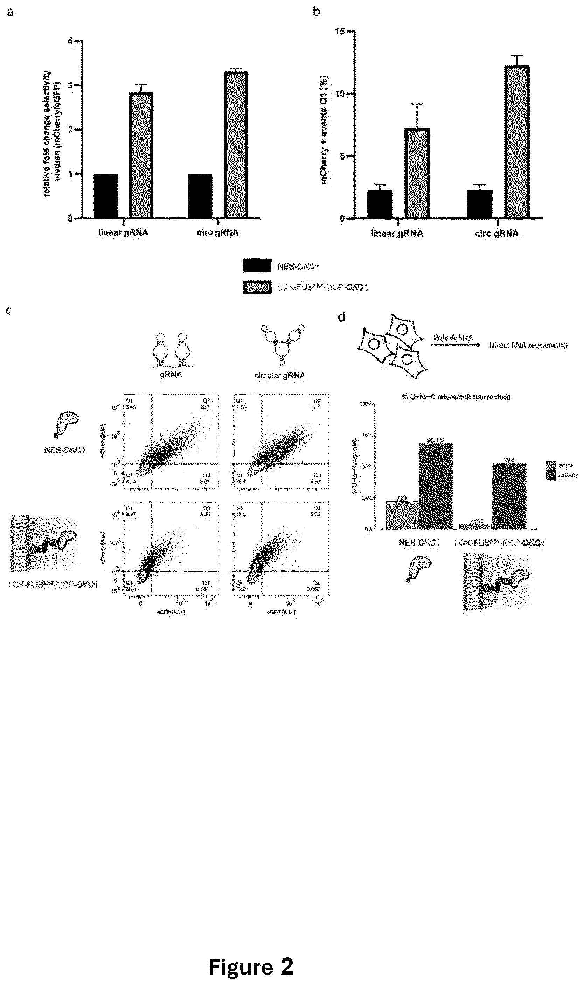

**Figure 2** illustrates that circular gRNAs increase designer organelle-mediated pseudouridylation. Figure 2a shows HEK293T cells were co-transfected with the selectivity reporter, gRNAs, and either NES_DKC1 or LCK_FUS²⁻²⁶⁷_MCP_DKC1. 5 × 10⁴ live cells were measured and the relative fold change in selectivity was calculated for linear and circular gRNAs and normalized to NES-DKC1. Figure 2b, simultaneously, cells were gated and analyzed for mCherry + cells. SEMs of three independent replicates for Figure 2a and Figure 2b are shown. Figure 2c, representative flow cytometry scatter plots shown for Figure 2a and Figure 2b. Figure 2d, U-to-C mismatches [%] at the desired target sites in the mCherry and eGFP reporter are shown. Similar to the flow cytometry data in Figure 2c, one observes a lower abundance of both reporters (GFP and mCherry) in the OREO experiments, which can likely originate from lower mRNA or protein levels or both. However, gratifyingly, the fold change in GFP mismatch is markedly higher (22%/3.2% ~ 6 fold) compared to detected mismatches in mCherry in the OREO conditions (68.1%/52% ~ 1 fold), showing that in the OREO containing experiments, modification of cytoplasmic mRNA (eGFP reporter), i.e. unwanted modification of U to Ψ, is reduced, while modification of mRNA targeted to the OREO (mCherry) stays efficient.

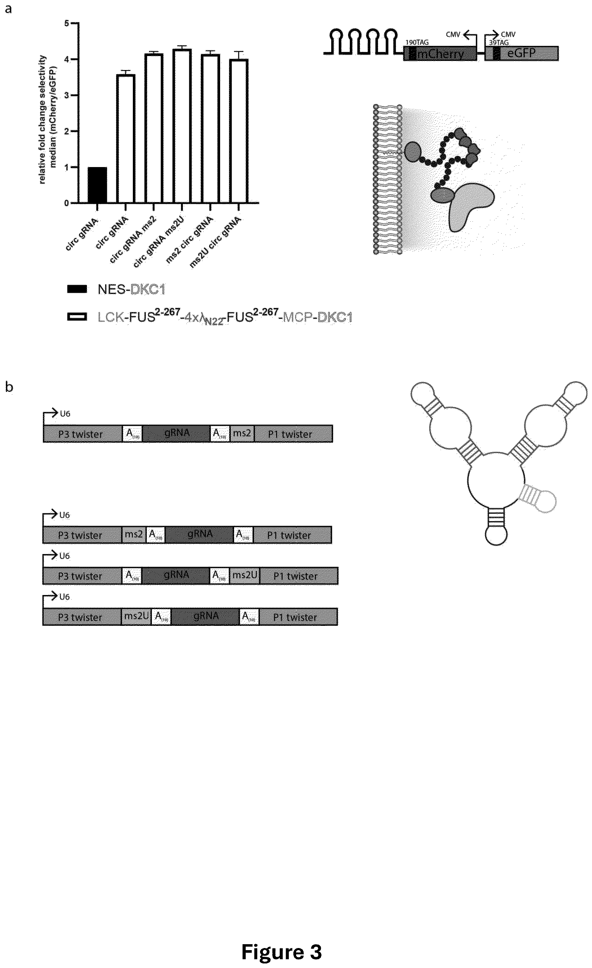

**Figure 3** illustrates circular gRNA tagged with an ms2 loop enhance selectivity. Figure 3a, HEK293T cells have been transfected with a modified selectivity reporter in which mCherry has been tagged with four boxB loops, DKC1 constructs where 4xλN22 peptides have been added separated from the MCP domain by another FUS²⁻²⁶⁷, and circular gRNAs for eGFP39TAG and mCherry^{190TAG_4xboxB}. The boxB loops in this modified selectivity reporter can bind to 4xλN22 peptides within the DKC1 organelle construct. Figure 3b, the circular gRNAs of mCherry include versions of ms2 aptamer loops 5' or 3' of the gRNA sequence, circ gRNA constructs used are shown, ms2U corresponds to a version of the ms2 apatmer with a lower affinity to MCP, an exemplary cartoon is shown for a circ gRNA having a 3' ms2 loop, the relative fold change in selectivity normalized to NES-DKC1 is plotted. SEMs from three independent experiments are shown.

The ability of pseudouridine to function as a stop-codon suppressor facilitates the development of a straightforward mRNA reporter that encodes for a fluorescent protein harboring a premature stop codon. Upon suppression of the stop codon, the mRNA can produce a fluorescent protein detectable via flow cytometry. A gRNA originating from H/ACA box RNA pugU2-34/44 of *Xenopus laevis,* which has been employed recently to mediate targeted stop-codon suppression, was designed for this purpose. The base pairs within the pseudouridylation pocket of the gRNA were customized to target an iRFP-eGF^{p39TAG} reporter, which would result in eGFP production only if pseudouridylation at the premature stop codon occurs.

The OREOs of the present invention are based on translating film-like organelles, comprising an N-terminal domain of the rodent LCK tyrosine kinase as a plasma membrane anchor, an intrinsically disordered region (IDR) derived from the protein FUS, and a major capsid protein (MCP), serving as a recruitment domain. These components, along with various control constructs lacking the LCK, IDR, or MCP domain, were N-terminally fused with dyskerin under the control of a CMV promoter. A truncated version of dyskerin (22-424), referred to hereafter as DKC1, which lacks NLS/NoLS signals at the N or C termini, was utilized, and a nuclear export signal was added at the N terminus.

To initially assess if such complex fusion constructs can still mediate stop-codon suppression through pseudouridylation, an iRFP-eGF^{p39TAG} reporter harboring two RNA stem loops derived from the ms2 phage in the 3' UTR, termed the efficiency reporter, was used. These RNA loops bind specifically to the MCP domain present in the organelle constructs, enabling selective recruitment of the efficiency reporter. HEK293T cells were then transiently transfected with the efficiency reporter, as well as different constructs of DKC1 and a custom-designed gRNA under the control of a U6 promoter. Subsequently, the cells were analyzed with flow cytometry, and the percentage of GFP-positive cells was calculated, indicating successful stop-codon pseudouridylation. An LCK-FUS²⁻²⁶⁷-MCP-DKC1 fusion construct, in which the efficiency reporter is actively recruited via the ms2/MCP tagging system, mediated efficient nonsense suppression when co-transfected with a gRNA, albeit with lower efficiency than NES-DKC1. Constructs lacking either the IDR or MCP demonstrated lower efficiency in comparison. Slightly higher efficiency in DKC1 constructs harboring FUS²⁻²⁶⁷, hinting towards an enhancing effect of the film-like organelle design was observed.

Having established the functionality of the OREO construct, it was confirmed whether the organelle can mediate selective stop-codon pseudouridylation. For this, a bidirectional expression plasmid encoding mCherry^{190TAG_2xms2} and eGFP^{39TAG}, where both reporters are transcribed separately under the control of a CMV promoter, was used. HEK293T cells were then transfected with the organelle construct and two specific gRNAs targeting the premature stop codons in the fluorescent reporters. In this scenario, mCherry fluorescence was anticipated to dominate in cells co-transfected with the plasma membrane organelle, as the mCherry reporter mRNA is actively recruited via the ms2/MCP tagging system. The relative fold change of selectivity, defined as the ratio of the median mCherry and eGFP intensities in the flow cytometric analysis, was calculated. A more than 2.5-fold increase in selectivity was observed in cells hosting the designer organelle compared to cells expressing NES-DKC1. Conversely, inverting the reporter design (eGFP^{39TAG_2xms2}/mCherry) qualitatively inverted this effect.

### Circular gRNAs markedly enhanced pseudouridine-mediated stop-codon suppression

Following the setup, efforts to optimize the efficiency of the organelle for pseudouridine-dependent stop-codon suppression were inspired by an elegant approach for the circularization of small aptamers. The chosen method involved expressing the gRNA, targeting the efficiency reporter, under the control of a U6 promoter flanked by twister ribozymes and a 10-nt polyadenine linker (Figure 1a). After expression, twister ribozymes self-cleaved, leaving an RNA stem that was subsequently ligated by the endogenous RNA ligase RtcB. The circularization of the gRNAs was confirmed by RT-PCR, where only a circular gRNA results in a PCR product (Figure 1d). Excitingly, circular gRNAs increased the population of GFP-positive cells markedly, affording up to 10% more GFP-expressing cells in the case of the organelle of the invention (Fig. 1b). It is suspected that the increase arises from the greater resistance of circular gRNAs to exonucleases, allowing them to accumulate at much higher levels than their linear counterparts.

Further engineering was speculated upon for circular gRNAs, perhaps also for their active recruitment to the OREOs. In this vein, different versions of ms2 loops were added 5' and 3' of the gRNA (Figure 3). In parallel, LCK-FUS²⁻²⁶⁷-4x_{λN22}-FUS²⁻²⁶⁷-MCP-DKC1 organelles were designed, which comprise four repeats of the λN22 peptide, facilitating the recruitment of RNAs tagged with boxB RNA loops. Additionally, another FUS²⁻²⁶⁷ linker was inserted between the two RNA-binding proteins, aiming to retain flexibility and allow full access to both recruitment domains. Because the circular guide RNAs were tagged with MS2 loops, a switch to a bidirectional mCherry^{190TAG_4xboxB}/eGFP^{39TAG} reporter was made to avoid competition between circular gRNAs and the reporter for MCP binding. After comparing cells co-transfected with ms2 tagged to unlabeled circular gRNAs, a further optimization in selectivity up to 4-fold was observed when using these OREOs (Figure 3).

### Selective pseudouridylation with circular gRNAs

In an ongoing effort to demonstrate the effectiveness of circular gRNAs, their impact was tested in a dual-color reporter experiment previously introduced. HEK293T cells were transfected with the selectivity reporter and circular gRNAs for mCherry^{190TAG_2xms2} and eGFP^{39TAG}, along with the OREO. These organelles maintained high selectivity, preferentially producing mCherry. The enhancing effect of circular gRNAs became more apparent when considering the percentage of mCherry-positive cells, which more than doubled on average compared to OREOs using linear gRNAs (Figure 2b, c).

To validate RNA pseudouridylation qualitatively but directly, direct RNA-sequencing (DRS; Oxford Nanopore Technologies) was employed. DRS allows for the sequencing of natural RNA without prior amplification steps. Specific current changes during the sequencing process were detected and used to identify the underlying canonical bases. It has been shown that DNA or RNA base modifications can differ in their current signal pattern from their canonical counterparts, thereby facilitating the identification of such modifications.

For pseudouridine modifications, the most commonly observed pattern is a systematic uridine-cytosine mismatch, i.e. pseudouridine is detected as a C 20,21. This facilitates the classification of individual reads into unmodified and modified reads based on basecalling error information, allowing quantification of pseudouridine modifications at specific sites. PolyA RNA was extracted from cells used in a selectivity assay (Figure 2c) and from cells in which a nontargeting circular gRNA was expressed with the organelle and selectivity reporter, followed by the direct RNA-sequencing protocol from Oxford Nanopore Technologies. After mapping the basecalled reads onto the eGFP and mCherry reporter sequences, the basecalling error patterns, including U-C mismatches, were extracted for all reads mapping on the mCherry and EGFP reporter sequence from both the NES-DKC1- and designer organelle-transfected cells. The method is rather qualitative then quantitative. However, relative changes are consistent with the flow cytometry data, that is, OREO-targeted mCherry is preferentially pseudouridylated at the desired position (Figure 2d).

### Methods

### Cloning

Constructs in this work were cloned using either restriction ligation, golden gate assembly or gibson assembly. The efficiency and selectivity reporters have been previously created¹². DKC1 constructs were assembled in pcDNA3.1 backbone via Gibson assembly. Guide RNAs were constructed by amplifying via forward and reverse primers and cloned into a puc57 backbone under the control of a U6 promoter using golden gate assembly. Circular guide RNAs were cloned into a puc57 backbone containing P3 twister and P1 twister ribozymes using golden gate assembly.

### Cell culture

HEK293T cells (ATCC CRL-3216) were maintained in Dulbecco's modified Eagle's medium (DMEM, Gibco 41965-039) supplemented with 1% penicillin-streptomycin (Sigma-Aldrich P0781), 1% L-Glutamine (Sigma-Aldrich G7513), 1% sodium pyruvate (Life Technologies 11360), and 10% FBS (Sigma-Aldrich F7524). Cells were cultured at 37°C in a 5% CO₂ atmosphere and passaged every 2-3 days up to 20 passages.

### Flow cytometry

1.1 × 10⁵ HEK293T cells were seeded in 24well plates and transfected roughly 17h later using jetPrime (Polyplus) as indicated by the manufacturer. Plasmids for organelle/gRNA/reporter constructs were transfected in a 1:1:1 ratio for experiments in Fig1A with a total of 1.2ug DNA per well. The media was exchanged after 4h and cells were grown for additional 48h. For flow cytometry measurements cells were washed once with PBS and trypsinized. Cells were resuspended in 900ul resuspension buffer 1 (1x PBS, 10% FBS, 2mM sodium azide, 2mM EDTA) and centrifuged for 5min at 400g at 4C. Supernatant was poured off and cells were resuspended in 900ul resuspension buffer 2 (1x PBS, 3% BSA, 2mM sodium azide, 2mM EDTA) and centrifuged again for 5min at 400g and 4C. Supernatant was again poured off and cells were resuspended in 300ul resuspension buffer 2 and cooled on ice until the measurement. Data was collected with a LSRFortessa SORP (BD Biosciences). DAPI (50 µg/mL) was added before measurement to distinguish live from dead cells. 1.1 × 10⁵ live cells were measured for each sample. Data was analyzed using FlowJo (v10.7.1.) HEK293T cells were distinguished based on FSC-A & SSC-A. Single cells were gated based on SSC-W & SSC-A. Live cells were distinguished based on SSC-W & 405-450/50 channel. For experiments using the efficiency reporter cells were then divided in four quadrants based on the reporter only control (Fig. S5). To estimate transfection efficiency from iRFP expressing cells cells were gated based on SSC-F & 640-730/45 channel. Final GFP + percentages (Q2) were calculated by normalizing to iRFP + percentages of NES-DKC1. Normalized GFP + percentages (Q2) have been plotted in Figure 1b & 3c. Data was plotted using Graph pad Prism software (v9.1.1). For experiments using the selectivity reporter eGFP fluorescence was acquired using a 488 nm laser and a 530/30 bandpass filter and for mCherry, a 561 nm laser with a 610/20 bandpass filter. Cells were gated similar to efficiency reporter experiments and quadrants were adjusted to a reporter only control in Q4. For analysis of selectivity experiments a NOT gate for Q4 was created in FlowJo to collect total eGFP and mCherry intensities from Q1-Q3. Relative fold changes in selectivity were calculated by dividing median mCherry intensities by median eGFP intensities. Calculated fold changes were normalized to NES-DKC1.

### Microscopy

5.5 × 10⁴ HEK293T cells were seeded in 8 well ibidi dishes coated with poly-l lysine. Cells were transfected after 17h using PEI. Therefore, 600µg of total DNA were added to 25ul DMEM-PEI. DNA:PEI ratio was 1:3. After 48h cells were washed with PBS and then fixed for 10min at RT in 2% paraformaldehyde. Cells were once washed 2x with PBS. Cells were permeabilized with 0.5% Trition-X-100 in PBS for 5min at RT and then washed 2x with PBS. Blocking solution (1% donkey serum in PBS/0.1% Tween-20) was added for 10min at RT. First antibody (mouse anti-HA 1:500, gift from Dorothee Dormann) in blocking solution was added overnight at 4 degrees. Cells were then washed 3x with PBS/0.1% Tween-20 for 5-10min. Second antibody (donkey anti-mouse conjugated with Alexa Fluor^{™} 555, A-31570 Thermo Fisher Scientific) was added in blocking buffer for 1h at RT. Cells were again washed 3x with PBS/0.1% Tween for 5-10min and then stained with DAPI (0.5ug/ml, 1:2000) in PBS for 5min at RT. Afterwards cells were washed 2x with PBS and kept at 4 degree until imaging. Cells were imaged using a BC43 Andor benchtop confocal microscope with a 100x oil objective. Images were processed using Fiji.

### RNA extraction

1.5 × 10⁶ HEK293T cells were seeded in P10 dishes and transfected 17-18h later. For transfection 48ul of jetPrime Reagent were mixed with 24µg total DNA (plasmid were kept 1:1:1 ratio) in 1ml jetPrime Buffer. Medium was exchanged after 4h and cells were grown for 48h. Cells were washed 1x with PBS and then trypsinized. After neutralization with DMEM, cells were collected in 15ml centrifugation tubes. Cells were centrifuged for 5min at 400g at 4 degrees. Supernatant was poured off and cells were resuspended in 2ml PBS. Cells were centrifuged again for 5min at 400g at 4 degrees. Supernatant was poured off and the cell pellet was flash frozen in liquid nitrogen and stored at -20 degree for further analysis. Poly-A RNA was isolated using mRNA Direct Kit (Invitrogen, 61012) according to the manufacturer's instructions. After two sequential rounds of oligo dT isolation poly-A RNA was eluted in 20µl Elution buffer supplied with the kit.

### Direct RNA Nanopore-seg library preparation

Library preparation was performed using Direct RNA Sequencing Kit (SQK-RNA002, ONT) using the manufacture's protocol (DRS_9080_v2_revS_14Aug2019). Briefly, 100 ng of poly(A)-tailed RNA was ligated to ONT RT Adaptor (RTA) using T4 DNA Ligase (NEB, M0202T) and was reverse transcribed using SuperScript III RT (Thermo Fisher Scientific, 18080044). The products were purified using 1.8X Agencourt RNAClean XP beads (Thermo Fisher Scientific, NC0068576) and RNA adapter (RMX) was ligated onto the RNA:DNA hybrid. The mix was purified using 1 × Agencourt RNAClean XP beads and washed twice using wash buffer (WSB). The sample was then eluted in elution buffer (EB) and mixed with RNA running buffer (RRB) before loading onto a primed R9.4.1 PromethlON flow cell.

### Bioinformatic analysis

Raw direct RNA sequencing data were basecalled using Dorado v0.3.2 underlying the high accuracy model for direct RNA sequenced on RNA002 flowcells (Dorado: Oxford Nanopore Technologies, Dorado.2023: https://github.com/nanoporetech/dorado) Basecalled reads were mapped onto the custom reference sequences from the eGFP and mCherry reporter using minimap2 v2.26 with the suggested parameters for direct RNA reads: -ax splice -uf -k14²⁶. The resulting bam files were indexed and filtered by samtools v1.16 using the -F 3488 flag (Samtools: https://doi.org/10.1093/gigascience/giab008) and a minimum read quality of 12 (=mean read quality of all samples) - extracting high quality reads only. Quality control of sequencing and mapping was done by NanoComp v1.23.1²⁷. Basecalling errors including mismatches, insertions and deletions were retrieved by samtools's mpileup function using the following parameters: -Q12 -x -no-output-ins -no-output-del -no-outut-ends. The resulting mpileup table was further processed by a custom R script to extract the percentages of different basecalling patterns including match, mismatch, deletion and insertion using R v4.2.2 and Rstudio v2022.7.2.576 (https://www.R-project.org/., http://www.rstudio.com/). For pseudouridine modifications, Uridine-to-Cytosine (U-to-C) mismatch percentages were calculated to the positions of interest in the mCherry and eGFP reporters. To remove the standard error pattern for the specific sequence region, the percentage of U-to-C mismatches found in the unmodified control sample on the same sites was substracted from the resulting U-to-C mismatch amounts of the reporters, respectively. For accuracy metrics, the match and mismatch percentages for each base of the sequences is plotted as heatmap using the R package pheatmap (version 1.0.12, https://CRAN.R-project.org/package=pheatmap). All other plots are created by using the R package ggplot2.

## Claims

1. A nucleic acid molecule complex for pseudouridylation of a target RNA in a mammalian cell comprising a guide RNA (gRNA) that at one end is flanked by a first twister ribozyme and at its other end by a second twister ribozyme, wherein the nucleic acid molecule complex has the capability to generate circular gRNA which is **characterized by** a secondary structure comprising two pseudouridylation pockets that harbor antisense guide sequences.

2. The nucleic acid molecule complex according to claim 1, wherein the first twister ribozyme is a P3 twister ribozyme and the second twister ribozyme is a P1 twister ribozyme, wherein each twister ribozyme is separated from the gRNA by a linker.

3. The nucleic acid molecule complex according to claim 2, wherein the linker is a poly(A) linker, preferably comprising between 5 and 20 nucleotides.

4. The nucleic acid molecule complex according to any one of claims 2 to 3, wherein the nucleic acid molecule complex further comprises a promoter, preferably a U6 promoter, to express a 5' - P3 twister ribozyme - gRNA - P1 twister - 3' molecule.

5. The nucleic acid molecule complex according to any one of claims 1 to 4, wherein the gRNA is H/ACA box small nucleolar RNA (H/ACA snoRNA).

6. The nucleic acid molecule complex according to claims 1 to 5, wherein the nucleic acid molecule complex further comprises one or more short aptamer sequences of RNA loops that are arranged after the first twister ribozyme and/or before the second twister ribozyme.

7. The nucleic acid molecule complex according to claim 6, wherein the short aptamer sequences of RNA loops sequences are selected from the group consisting of a ms2 loop, boxb, pp7 or Qbeta loop.

8. The nucleic acid molecule complex according to claim 6 or claim 7, wherein the nucleic acid molecule complex interacts via the short aptamer sequences with an optimized RNA editing organelle (OREO) comprising
a. a plasma membrane anchor,
b. an intrinsically disordered region (IDR), and
c. a major capsid protein (MCP) serving as a recruitment domain for the nucleic acid molecule complex.

9. The nucleic acid molecule complex according to claim 8, wherein the MCP of the OREO is N-terminally fused to pseudouridine synthase dyskerin, preferably to a truncated version of dyskerin (22-424).

10. The nucleic acid molecule complex according to claims 8 or 9, wherein the plasma membrane anchor of the OREO consists of a N-terminal domain of LCK tyrosine kinase and the IDR is derived from the protein FUS.

11. A method for converting a uridine in a target RNA molecule into a pseudouridine, comprising the steps of:
- providing a target RNA molecule containing at least one uridine residue,
- providing a pseudouridine synthase enzyme,
- synthesizing a circular gRNA that includes sequences complementary to regions flanking the uridine residue in the target RNA,
- incubating the target RNA with the circular gRNA and the pseudouridine synthase under conditions suitable for binding and modification,
- and detecting the conversion of uridine to pseudouridine using mass spectrometry or nucleoside analysis,
wherein the circular gRNA used in the method is generated by using a nucleic acid molecule complex as defined in any one of claims 1 to 10.

12. A nucleic acid molecule complex as defined in any one of claims 1 to 10 for use in the treatment of a genetic disease caused by premature stop codons.

13. The nucleic acid molecule complex for the use according to claim 12, wherein the genetic disease caused by premature stop codons is selected from the group consisting of cystic fibrosis, Duchenne muscular dystrophy (DMD), Beta-thalassemia, Hurler syndrome, Usher syndrome, Ataxia-Telangiectasia (AT), Retinitis pigmentosa.

14. A pharmaceutical composition, comprising a nucleic acid molecule complex as defined in any one of claims 1 to 10, and one or more of a pharmaceutically acceptable carrier, stabilizer or solvent.

15. A delivery vector for pseudouridylation of a target RNA in a mammalian cell, comprising a nucleic acid molecule complex as defined in any one of claims 1 to 10.

16. A mammalian cell, expressing a nucleic acid molecule complex as defined in any one of claims 1 to 10 or a delivery vector as defined in claim 15.

17. A method for generating circular gRNA, comprising the steps:
- providing a nucleic acid molecule complex as defined in any one of claims 1 to 10,
- transferring the nucleic acid molecule complex into a mammalian cell,
- expressing the nucleic acid molecule complex in said mammalian cell to produce circular gRNA.
